# EUROPÄISCHE PATENTSCHRIFT

(11) **EP 4 046 684 B1**
(45) Veröffentlichungstag und Bekanntmachung des Hinweises auf die Patenterteilung: **23.08.2023**
(21) Anmeldenummer: 21158631.8
(22) Anmeldetag: 23.02.2021
(51) Int. Cl.: A61N 5/06

(54) **VORRICHTUNG ZUM BEHANDELN VON BIOLOGISCHEM GEWEBE**
BIOLOGICAL TISSUE TREATMENT APPARATUS
DISPOSITIF DE TRAITEMENT DES TISSUS BIOLOGIQUES

(43) Veröffentlichungstag der Anmeldung: 24.08.2022
(73) Patentinhaber: Medizinisches Laserzentrum Lübeck GmbH, 23562 Lübeck (DE)
(72) Erfinder: Brinkmann, Ralf, 23562 Lübeck (DE)
(74) Vertreter: Glawe, Delfs, Moll

(56) Entgegenhaltungen:
- EP-A1- 1 279 385
- WO-A1-2010/028822
- WO-A2-2018/209118
- DE-A1-102010 018 679
- US-B2- 10 806 513
- INGO ULRIK TEUDT ET AL: "Acoustic Events and Optophonic Cochlear Responses Induced by Pulsed Near-Infrared LASER", IEEE TRANSACTIONS ON BIOMEDICAL ENGINEERING, IEEE SERVICE CENTER, PISCATAWAY, NJ, USA, Bd. 58, Nr. 6, 1. Juni 2011 (2011-06-01), Seiten 1648-1655, XP011408389, ISSN: 0018-9294, DOI: 10.1109/TBME.2011.2108297
- YANG MUQUN ET AL: "Photoacoustic effect invokes auditory response in infrared neuron stimulation", JOURNAL OF INNOVATIVE OPTICAL HEALTH SCIENCES, [Online] Bd. 12, Nr. 01, 1. Januar 2019 (2019-01-01), Seite 1850040, XP055829896, Singapore ISSN: 1793-5458, DOI: 10.1142/S1793545818500402 Gefunden im Internet: URL:https://www.worldscientific.com/doi/pd f/10.1142/S1793545818500402> [gefunden am 2021-08-03]

## Beschreibung

Die Erfindung betrifft eine Vorrichtung zum Behandeln von biologischem Gewebe.

Es ist bekannt, dass sich eine therapeutische Wirkung einstellen kann, wenn Gewebe mit einer Frequenz der Schwingung im Ultraschallbereich in Schwingung versetzt wird. Beschrieben wurde dies u.v.a. für Nervenzellen, Teudt et al., IEEE T Biomed Eng 58(6) 2011, sowie für Gehörnerven der Cochlea, Wenzel et al., JBO 14(4) 2009.

Gewebe kann in Schwingung versetzt werden, indem mit repetierenden Lichtpulsen, die vom Gewebe absorbiert werden, auf das Gewebe eingewirkt wird. Die Lichtpulse lösen eine thermoelastische Expansion und anschließende Kontraktion aus und können deswegen zum Anregen der Schwingung verwendet werden. Als nicht ganz einfach hat es sich bislang herausgestellt, die Lichtquelle so einzustellen, dass sich in dem biologischen Gewebe die gewünschten Wirkungen einstellen, ohne das Gewebe zu schädigen. Wie intensiv das Gewebe auf Lichtpulse reagiert, hängt von der Art und vom Zustand des Gewebes sowie von der Amplitude und Frequenz der Schwingung ab. Bei Gewebe, das intensiv auf die Lichtpulse anspricht, besteht die Gefahr, dass das Gewebe durch eine zu hohe Amplitude dauerhaft geschädigt wird, wenn die Lichtpulse zu stark sind. Bei weniger empfindlichem Gewebe kann es sein, dass die gewünschten Wirkungen sich nicht einstellen, wenn die Lichtpulse zu schwach oder die Amplituden zu niedrig sind.

Dokument WO 2010/028822 A1 beschreibt eine Vorrichtung zum Behandeln von biologischem Gewebe mit einer Lichtquelle und mit einem Schwingungssensor. Die Messungen des Schwingungssensors werden analysiert und zur Steuerung der Lichtquelle benutzt. Die Analyse basiert auf einer Abweichung eines gemittelten Verlaufs der gemessenen Druckamplituden von einer vorab bekannten Funktion.

Der Erfindung liegt die Aufgabe zugrunde, eine Vorrichtung zum Behandeln von biologischem Gewebe vorzustellen, bei der das Gewebe mit gepulstem Licht behandelt werden kann, so dass sich zuverlässig ein gewünschter Schwingungszustand des Gewebes einstellt. Ausgehend vom genannten Stand der Technik wird die Aufgabe gelöst mit den Merkmalen des unabhängigen Anspruchs. Vorteilhafte Ausführungsformen sind in den Unteransprüchen angegeben.

Die erfindungsgemäße Vorrichtung umfasst eine Lichtquelle, um innerhalb eines Behandlungszeitraums eine Vielzahl von Lichtpulsen auf das Gewebe zu senden, so dass das Gewebe in Schwingung versetzt wird. Die Vorrichtung umfasst weiter einen Schwingungssensor, der die Amplitude einer durch die Lichtquelle ausgelösten Schwingung des Gewebes misst. In einer Steuereinheit wird ein Relativwert gebildet, indem ein aktueller Messwert der Amplitude mit einem Anfangsmesswert der Amplitude in ein Verhältnis gesetzt wird. Der Relativwert wird in der Steuereinheit verarbeitet, um ein Steuersignal für die Lichtquelle zu erzeugen.

Die Erfindung beruht auf dem Gedanken, die Lichtquelle anhand eines Steuersignals anzusteuern, das basierend auf einer tatsächlich durch einen Lichtpuls ausgelösten Schwingung des Gewebes ermittelt wurde. Die Lichtquelle wird also nicht länger anhand von vorab getroffenen Annahmen über die Wirkung der Lichtpulse auf das Gewebe eingestellt, sondern es wird ein Steuersignal für die Lichtquelle erzeugt, das von der tatsächlichen Schwingung des Gewebes abhängt. Damit wird die Möglichkeit geschaffen, die Lichtquelle gezielt so einzustellen, dass sich in dem Gewebe bestimmte Schwingungszustände einstellen.

Es wurde erkannt, dass der absolute Wert der gemessenen Amplitude für sich genommen nicht immer ein geeignetes Kriterium für die Ansteuerung der Lichtquelle darstellt. Mit der Erfindung wird deswegen vorgeschlagen, einen aktuellen Messwert der Amplitude in ein Verhältnis zu setzen zu einem Anfangsmesswert der Amplitude. Der zu Beginn des Behandlungszeitraums aufgenommene Anfangsmesswert gibt an, wie das Gewebe zu Beginn der Behandlung auf die Lichtpulse reagiert. Das Verhältnis zwischen dem aktuellen Messwert und dem Anfangsmesswert der Amplitude bildet ein Maß, inwieweit das Gewebe in Schwingung versetzt wird. In Form des Relativwerts wird diese Information in der Steuereinheit verarbeitet, um den weiteren Gang der Behandlung mit Lichtpulsen zu steuern.

Die Amplitude der Gewebeschwingung hängt von der Pulsenergie ab, je höher die Pulsenergie desto größer wird die Amplitude. Wird die Amplitude der Gewebeschwingung größer, obwohl die Pulsenergie gleich bleibt, so deutet dies auf stärkere Schwingung hin.

Der Anfangsmesswert und der aktuelle Messwert der Amplitude können auf die Pulsenergie normiert werden, so dass der Relativwert ein normierter Relativwert ist. Aus dem normierten Relativwert kann abgelesen werden, ob die Amplitude der Gewebeschwingung bezogen auf dieselbe Pulsenergie sich verändert hat. Der Relativwert ist automatisch ein normierter Relativwert in diesem Sinne, wenn Lichtpulse mit identischer Pulsenergie auf das Gewebe gesendet werden.

Die Behandlung mit der erfindungsgemäßen Vorrichtung dient nicht dazu, eine dauerhafte Strukturveränderung des Gewebes herbeizuführen. Vielmehr soll das Gewebe durch die Schwingung angeregt oder stimuliert werden, ohne dass das Gewebe in seiner Struktur geschädigt wird. Bei zu starkem Anstieg der Amplitude besteht ein Risiko einer mechanischen Schädigung des Gewebes durch Aufreißen der Strukturen oder einer thermischen Schädigung durch nicht mehr ausreichende Dissipation der entstehenden Wärme. Es kann deswegen ein Grenzwert für den Relativwert vorgegeben werden, und die Lichtquelle kann so angesteuert werden, dass der Relativwert den Grenzwert nicht überschreitet. Der Grenzwert kann beispielsweise einen Wert zwischen 1,1 und 2, vorzugsweise zwischen 1,3 und 1,6 haben.

Möglich ist auch, einen Zielwert für den Relativwert vorzugeben und die Lichtquelle so anzusteuern, dass der Relativwert den Zielwert erreicht. Bei Erreichen des Zielwerts kann die Behandlung beendet werden, die Lichtquelle also so angesteuert werden, dass sie keine Lichtpulse mehr abgibt. Möglich ist auch, die Lichtquelle so anzusteuern, dass der Relativwert für eine vorgegebene Zeitspanne bei dem Zielwert bleibt. Auch andere Varianten sind möglich, beispielsweise, dass die Behandlung nach Erreichen des Zielwerts mit einem niedrigeren Relativwert fortgesetzt wird. Dies kann beispielsweise erreicht werden, indem die Pulsenergie oder Pulsdauer der Lichtquelle reduziert wird und/oder der zeitliche Abstand zwischen zwei aufeinander folgenden Pulsen vergrößert wird. Der Zielwert für den Relativwert kann beispielsweise zwischen 1,05 und 1,5, vorzugsweise zwischen 1,1 und 1,3, weiter vorzugsweise zwischen 1,15 und 1,25 liegen.

In einer Ausführungsform ist eine Kennlinie über der Zeit für den Relativwert vorgegeben und die Lichtquelle wird so angesteuert, dass der Relativwert der Kennlinie folgt.

Die Lichtquelle kann so angesteuert werden, dass Lichtpulse mit gleichbleibender Energie abgegeben werden. Der zeitliche Abstand der Lichtpulse während einer Behandlung kann gleich bleiben. Möglich ist auch, den zeitlichen Abstand innerhalb einer Behandlung zu verändern, um den Verlauf der Gewebeschwingung zu beeinflussen.

In einer anderen Ausführungsform wird zu Beginn der Behandlung ein Lichtpuls von niedriger Energie auf das Gewebe gesendet, wobei die Energie so bemessen ist, dass eine Schädigung des Gewebes sicher ausgeschlossen ist. Im Verlauf der Behandlung kann die Pulsenergie gesteigert werden, bis sich ein gewünschter Schwingungszustand des Gewebes eingestellt hat. Anhand des normierten Relativwerts kann sichergestellt werden, dass die Amplitude nicht so weit ansteigt, dass das Gewebe geschädigt wird.

Die Vorrichtung kann einen geschlossenen Regelkreis umfassen, mit dem die Abgabe der Lichtpulse so geregelt wird, dass die Schwingung des Gewebes auf eine vorgegebene Amplitude eingestellt wird. Beispielsweise kann gewünscht sein, dass die Amplitude nach einem Anstieg zu Beginn der Behandlung auf einem konstanten Sollwert gehalten wird. Das Steuersignal an die Lichtquelle kann so gestaltet sein, dass der konstante Sollwert sich einstellt.

Möglich ist auch, dass eine Kennlinie für die Amplitude der Gewebeschwingung vorgegeben ist und dass die Lichtquelle mit dem geschlossenen Regelkreis so geregelt wird, dass die Amplitude gemäß der vorgegebenen Kennlinie eingestellt wird. Beispielsweise kann zu Beginn der Behandlung oder während eines Zeitabschnitts der Behandlung eine Kennlinie für einen vorgegebenen Anstieg oder Abfall der Amplitudenmesswerte über der Zeit vorgegeben sein. Die sich auf diese Weise ergebende Steuerung des Behandlungsverlaufs kann zusätzlich oder alternativ zu der auf den Relativwert bezogenen Steuerung erfolgen.

Die Lichtquelle kann so eingerichtet sein, dass für die Dauer einer Behandlung eine Vielzahl von gleichartigen Lichtpulsen auf das Gewebe gerichtet wird. Die Lichtpulse können gleiche Pulsdauer haben. Die Lichtpulse können eine gleiche Pulsenergie haben, so dass mit jedem Lichtpuls die gleiche Energiemenge auf das Gewebe übertragen wird. Möglich ist auch eine Behandlung mit voneinander abweichenden Lichtpulsen zur Anpassung an die Schwingung des Gewebes, wobei insbesondere die Pulsdauer und/oder die Pulsenergie und/oder die Pulsfolgefrequenz während der Dauer einer Behandlung von einer Steuereinheit variabel angesteuert werden können. Die Dauer eines einzelnen Lichtpulses kann beispielsweise zwischen 0,1 ns und 100 ps, vorzugsweise zwischen 10 ns und 2 µ.

Die Wellenlänge des Lichts kann ein, zwei oder mehr als zwei Spektralbereiche umfassen, die unterschiedlich stark vom Gewebe absorbiert werden, um das Gewebe in unterschiedlichen Abschnitten verschieden stark zum Schwingen anzuregen. Die Lichtquelle kann eine Laser-Lichtquelle sein, die Licht in einem eng begrenzten Spektralbereich abgibt. Die verschiedenen Spektralbereiche können dabei das gesamte Spektrum vom UV bis ins weite IR-Spektrum umfassen und beispielsweise zwischen 300 nm und 3000 nm liegen. In speziellen Ausführungsformen z.B. einen Laser mit Emission der Grundfrequenz und deren frequenzverdoppelter Komponente enthalten, z.B. 1064 nm und 532 nm. Pulse unterschiedlicher Wellenlänge können in beliebiger Reihenfolge synchron oder asynchron gemischt werden. Eine bevorzugte Variante zur Schwingungsanregung ist eine alternierende Abgabe von Lichtpulsen, wobei die Lichtpulse in verschiedenen Spektralbereichen liegen. Der zeitliche Pulsabstand kann gleich sein.

Die Lichtpulse können so abgegeben werden, dass die Dauer eines Lichtpulses kürzer ist als die Zeitspanne zwischen dem Ende eines ersten Lichtpulses und dem Beginn eines zweiten unmittelbar darauf folgenden Lichtpulses. Vorzugsweise ist die Dauer einer Pause zwischen zwei aufeinanderfolgenden Lichtpulsen um wenigstens den Faktor 10, vorzugsweise um den Faktor 100, weiter vorzugsweise um den Faktor 1000 länger als die Pulsdauer.

Die Pause zwischen zwei Lichtpulsen sollte nicht so lang sein, dass das Gewebe in dieser Zeit wieder auf seinen Ausgangszustand abklingt. Angestrebt wird vielmehr, dass das Gewebe durch die Summe der eingebrachten Lichtpulse zu einer kontinuierlichen Schwingung angeregt wird. Der Abstand der Pulse sollte daher vorzugsweise kürzer sein als die thermische Relaxationszeit des angeregten Gewebes.

Als eine Behandlung wird eine Folge von Lichtpulsen bezeichnet, die in Summe eine kontinuierliche Schwingungsbehandlung des Gewebes bewirken. Wird ein erneuter Lichtpuls auf das Gewebe gerichtet, nachdem das Gewebe zwischenzeitlich wieder zu seinem Ausgangszustand zurückgekehrt ist, so ist dies der Beginn einer neuen Behandlung. Die zeitliche Dauer einer Behandlung wird als Behandlungszeitraum bezeichnet.

Von der Erfindung umfasst ist auch eine Vorrichtung, die so eingerichtet ist, dass innerhalb des Behandlungszeitraums Lichtpulse in Form von Therapiepulsen und Lichtpulse in Form von Messpulsen auf das Gewebe gerichtet werden. Die Therapiepulse können eine andere räumliche Ausdehnung haben als die Messpulse. Andere räumliche Ausdehnung bedeutet, dass die auf dem behandelten Gewebe abgedeckte Fläche größer oder kleiner ist. Insbesondere kann die mit den Therapiepulsen abgedeckte Fläche größer sein als die mit den Messpulsen abgedeckte Fläche. Beispielsweise kann der Messpuls in der Mitte des Behandlungsareals platziert werden, um die Maximalamplitude der Gewebeschwingung zu erfassen. Zusätzlich oder alternativ zu der unterschiedlichen räumlichen Ausdehnung, können die Messpulse und die Therapiepulse auch unterschiedliche Pulsdauern und/oder Pulsfolgefrequenzen aufweisen.

Die Steuereinheit kann so eingerichtet sein, dass sie zum Zwecke der Ansteuerung der Lichtquelle nur Amplitudenmesswerte von durch Messpulse ausgelösten Schwingungen des Gewebes verarbeitet. Zwischen zwei Messpulsen können jeweils ein oder mehrere Therapiepulse eingeschlossen sein, deren Pulsdauer und Pulsenergie sich an den Erfordernissen der Behandlung orientieren.

Die Vorrichtung kann eine von der für die Behandlung des Gewebes verwendeten Lichtquelle separate Mess-Lichtquelle umfassen, um die Messpulse zu erzeugen. Die Messpulse können in einem anderen Spektralbereich liegen als die Therapiepulse. Möglich ist auch, dass die Messpulse und die Therapiepulse mit einer einheitlichen Lichtquelle erzeugt werden.

Die Lichtquelle kann eine Laser-Lichtquelle sein. Der Strahlengang der Lichtquelle kann so geformt sein, dass ein Spot des Gewebes beleuchtet wird. Die Spotgröße, also der Durchmesser des Spots kann in Abhängigkeit vom behandelten Gewebe gewählt werden. Wird die Retina des Auges behandelt, so kann die Spotgröße zwischen 50 µm und 500 µm, vorzugsweise zwischen 100 µm und 200 µm liegen. Beim Trommelfell des Ohres kann beispielsweise der Spot 5 mm groß sein. Bei anderen Arten von Geweben wie beispielsweise subkutanem Nervengewebe kann mit anderen Spotgrößen je nach Größe des Behandlungsareals gearbeitet werden, beispielsweise zwischen 2 mm und 20 mm, vorzugsweise zwischen 3 mm und 8 mm.

Die Bestrahlung eines einzelnen Lichtpulses kann beispielsweise zwischen 0,1 und 100 mJ/cm² liegen und weiter vorzugsweise kleiner sein als die nach der Europäischen Norm DIN-EN 60825-1 vorgegebene Maximalbestrahlung von Gewebe. Eine einzelne Behandlung kann zwischen 0,02 s und 100 s, vorzugsweise zwischen 0,05 s und 10 s, weiter vorzugsweise zwischen 0,1 s und 1 s dauern. Aber auch eine dauerhafte Schwingungsanregung beispielsweise zur modulierten Anregung des Trommelfells, Mittel- oder Innenohr ist denkbar. Die Anzahl der Lichtpulse während einer Behandlung kann beispielsweise zwischen 100 und 100.000 liegen. Als eine Behandlung wird eine Folge von Lichtpulsen bezeichnet, die in Summe eine kontinuierliche Schwingungsbehandlung des Gewebes bewirken. Wird ein erneuter Lichtpuls auf das Gewebe gerichtet, nachdem das Gewebe zwischenzeitlich wieder seinen Ausgangszustand eingenommen hat, so ist dies der Beginn einer neuen Behandlung. Alle Lichtpulse während einer Behandlung können auf denselben Bereich des Gewebes gerichtet werden.

Die Schwingung des Gewebes, die durch einen Lichtpuls ausgelöst wird, kann eine thermoelastische Expansion und Kontraktion sein. Der Schwingungssensor kann für eine direkte Messung der Gewebeexpansion ausgelegt sein. Diese ist beispielsweise möglich, wenn der Schwingungssensor ein optisches oder akustisches Interferometer ist.

Möglich ist auch, mit dem Schwingungssensor die bei der Schwingung abgestrahlte akustische Welle, deren Amplitude und/oder deren Spektrum zu messen und daraus einen Rückschluss auf den Schwingungszustand des Gewebes zu ziehen. Der Schwingungssensor kann ein Mikrophon, Hydrophon oder ein Piezo-Element umfassen, das auf die Druckwelle reagiert.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine Ausführungsform einer erfindungsgemäßen Vorrichtung;
- Fig. 2:: den Verlauf der durch einen Lichtpuls ausgelösten Schwingung zu Beginn einer Behandlung;
- Fig. 3:: die Ansicht aus Fig. 2 in einem späteren Stadium der Behandlung;
- Fig. 4-6:: alternative Ausführungsformen von erfindungsgemäßen Vorrichtungen;
- Fig. 7:: die Amplitude der Gewebeschwingung während des Verlaufs einer Behandlung.

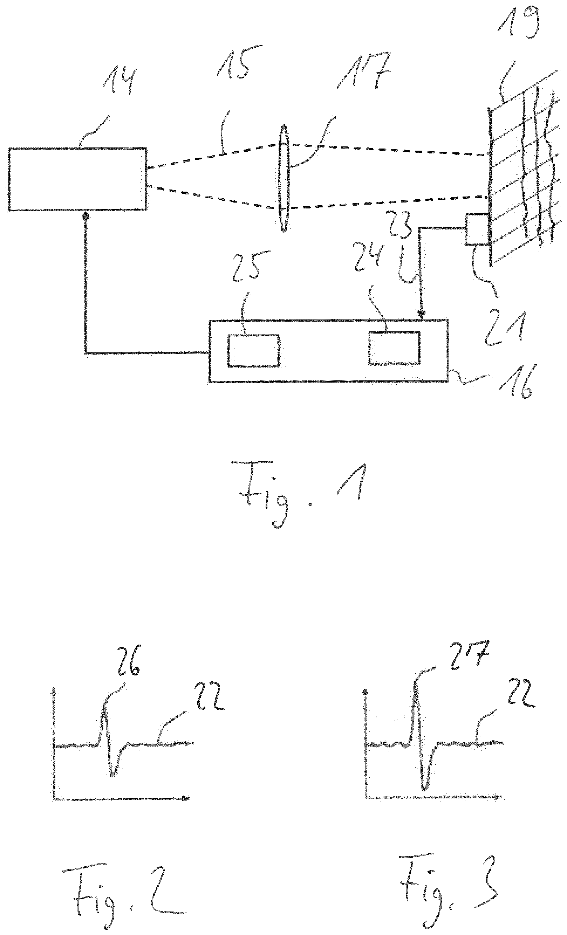

Eine in Fig. 1 gezeigte erfindungsgemäße Vorrichtung umfasst eine Laser-Lichtquelle 14, die unter der Kontrolle einer Steuereinheit 16 Lichtpulse 15 wenigstens einer Wellenläne abgibt. Die Lichtpulse 15 werden über geeignete optische Elemente 17 auf Nervengewebe 19 eines Patienten geleitet. Jeder Lichtpuls 15 induziert eine Schwingung des Retina-Gewebes in Form einer thermoelastischen Expansion. Die Schwingung löst eine Druckwelle 22 aus, die sich ausgehend von dem Nervengewebe 19 fortpflanzt. Die Fig. 2 und 3 zeigen beispielhafte Verläufe der Druckwelle über der Zeit.

Die Vorrichtung umfasst einen Schwingungssensor 21 in Form eines Hydrophons. Der Schwingungssensor 21 reagiert auf die Druckwelle 22 und gibt ein elektrisches Signal ab, das die Amplitude und die Frequenz der Druckwelle 22 repräsentiert. Die Amplitude der Druckwelle 22 korrespondiert mit der Amplitude der Schwingung des Nervengewebes 19.

Das von dem Schwingungssensor 21 erzeugte elektrische Signal wird als Amplitudenmesswert über eine Leitung 23 an die Steuereinheit 16 gesendet und in der Steuereinheit 16 verarbeitet. Zu Beginn einer Behandlung wird ein erster Lichtpuls 15 auf das Nervengewebe 19 gesendet, wodurch eine erste Druckwelle 22 ausgelöst wird, deren Amplitude in Fig. 2 gezeigt ist. Der Verlauf der Druckwelle 22 wird mit dem Schwingungssensor 21 registriert. Der zugehörige Amplitudenmesswert wird als Anfangsmesswert 26 der Amplitude in einem Speicher 24 gespeichert.

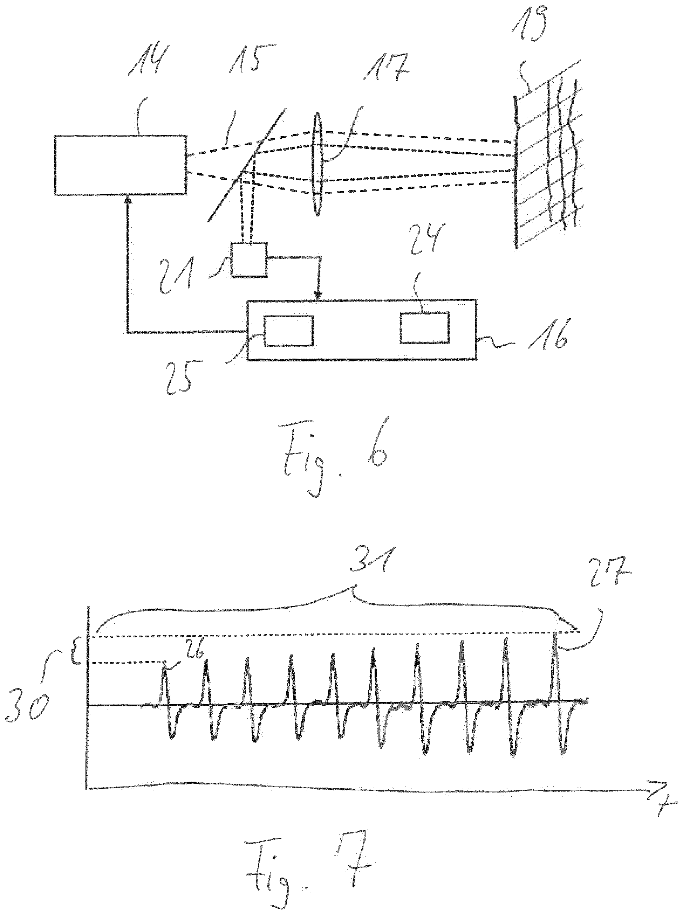

Während des Fortgangs der Behandlung werden in konstanter Taktung weitere gleichartige Lichtpulse 15 auf das Nervengewebe 19 geleitet. Jeder Lichtpuls 15 bewirkt eine erneute Anregung der Schwingung des Nervengewebes 19. In der Pause zwischen zwei Lichtpulsen 15 klingt die Schwingung des Nervengewebes 19 ab, ohne ganz auszuklingen. Die Lichtpulse 15 führen in Summe zu einer kontinuierlichen Schwingungsanregung des Nervengewebes 19. In Fig. 7 ist beispielhaft der zeitliche Verlauf einer Behandlung dargestellt, wobei die horizontale Achse die Zeit t und die vertikale Achse die Amplitude der Gewebeschwingung zeigt. Der erfindungsgemäße Relativwert 30 wird ermittelt, indem ein aktueller Messwert der Amplitude in ein Verhältnis zum Anfangsmesswert 26 der Amplitude gesetzt wird. Die Taktung der Lichtpulse ist so, dass die Gewebeschwingung während des Behandlungszeitraums 31 nie vollständig abklingt.

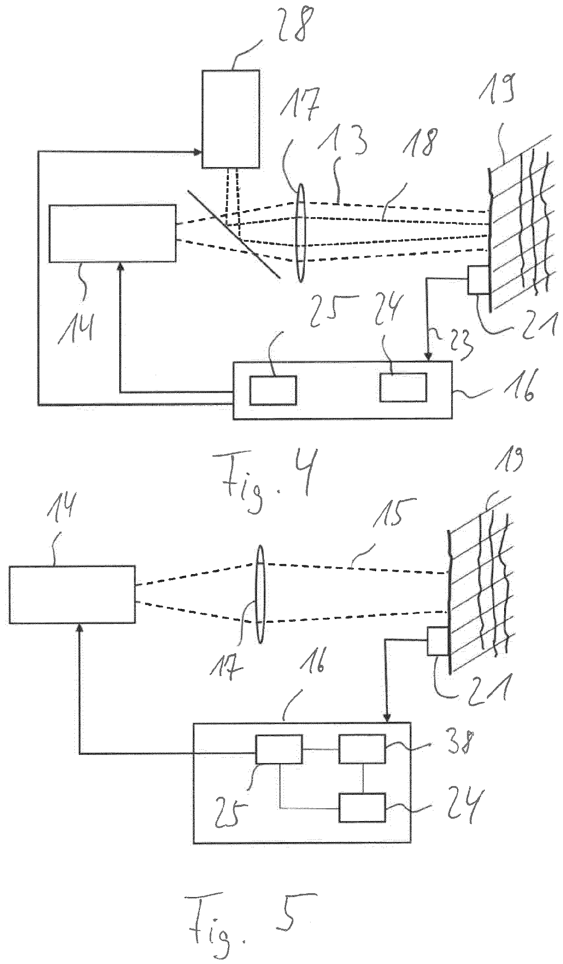

Durch die permanente Anregung mit gleichartigen Lichtpulsen 15 steigt die Amplitude der Druckwelle 22. In Fig. 3 ist die Druckwelle 22 am Ende der in Fig. 5 gezeigten Behandlung dargestellt. Der Amplitudenmesswert 27 ist um 50 % größer als der Anfangsmesswert 26 der Amplitude.

Alle während der Behandlung gewonnenen Amplitudenmesswerte 27 werden an die Steuereinheit 16 geleitet, wo ein Relativwert gebildet wird, indem jeder Amplitudenmesswert 27 in ein Verhältnis gesetzt wird zu dem in dem Speicher 24 hinterlegten Anfangsmesswert 26 der Amplitude. Der Relativwert wird in einem Komparator 25 der Steuereinheit 16 mit einem Grenzwert verglichen. Sobald der Relativwert den Grenzwert erreicht, sendet die Steuereinheit 16 ein Abschaltsignal an die Lichtquelle 14, so dass die Lichtquelle 14 keine Lichtpulse 15 mehr abgibt. Der Grenzwert für das Verhältnis zwischen dem Amplitudenmesswert und dem Anfangsmesswert 26 der Amplitude (Relativwert) liegt in dem Ausführungsbeispiel bei 1,5, der Grenzwert wird also erreicht, wenn der Amplitudenmesswert 27 um 50 % höher liegt als der Anfangsmesswert 26 der Amplitude. In Fig. 7 wird die Behandlung bei Erreichen des Grenzwerts beendet. Das Nervengewebe nimmt daraufhin wieder seinen schwingungslosen Ausgangszustand an.

Bei dem Ausführungsbeispiel gemäß Fig. 4 sendet die Lichtquelle 14 bevorzugt keine gleichartigen Lichtpulse 15 aus, sondern die Pulsenergie und/oder die Taktung ändern sich während der Dauer der Behandlung. Die Vorrichtung umfasst eine zusätzliche Mess-Lichtquelle 28, die in den Pausen zwischen den von der Lichtquelle 14 abgegebenen Therapiepulsen 13 Messpulse 18 auf das Nervengewebe 19 leitet. Die Therapiepulse 13 decken auf dem Nervengewebe 19 eine größere Fläche ab als die Messpulse 18. Nur die von den Messpulsen 18 ausgelösten Druckwellen werden in der Steuereinheit 16 ausgewertet, und mit dem ebenfalls mit einem Messpuls 18 gewonnenen Anfangsmesswert 26 der Amplitude in ein Verhältnis gesetzt. Auf diese Weise kann der Verlauf der Therapiepulse 13 sich an den Bedürfnissen der Behandlung ausrichten, ohne dass die Messung mit den Messpulsen 18 beeinträchtigt wird. Nach Erreichen eines Zielwerts für die Amplitude wird die Pulsenergie der Therapiepulse 13 vermindert, so dass die Amplitude der Schwingung des Nervengewebes 19 für die verbleibende Dauer der Behandlung konstant bleibt.

Bei der Ausführungsform gemäß Fig. 5 werden ebenfalls die Lichtpulse 15 während der Dauer der Behandlung variiert. In dem Speicher 24 der Steuereinheit 16 wird nicht nur der Anfangsmesswert 26 der Amplitude gespeichert, sondern auch die Pulsenergie des ersten Lichtpulses 15, mit dem die betreffende Druckwelle 22 ausgelöst wurde. Nachfolgende Druckwellen werden ausgelöst mit Lichtpulsen 15, die eine andere Pulsenergie, Pulsdauer und/oder Pulsfolgerate haben. Bevor der Amplitudenwert 27 dem Komparator 25 für den Vergleich mit dem Anfangsmesswert 26 der Amplitude zugeführt wird, wird in dem Baustein 38 der Amplitudenwert 27 auf die Pulsenergie normiert. Es wird ein normierter Relativwert erzeugt, der das auf die Pulsenergie normierte Verhältnis des Amplitudenwerts 27 zum Anfangsmesswert 26 der Amplitude wiedergibt und der eine Veränderung des Zustands des Nervengewebes 19 repräsentiert.

In Fig. 6 ist eine alternative Ausführungsform dargestellt, in der die Amplitude der Gewebeschwingung mit einem Schwingungssensor 21 in Form eines optischen Sensors gemessen wird.

## Patentansprüche

1. Vorrichtung zum Behandeln von biologischem Gewebe (19), mit einer Lichtquelle (14), um innerhalb eines Behandlungszeitraums (31) eine Vielzahl von Lichtpulsen (15) auf das Gewebe (19) zu senden, so dass das Gewebe (19) in Schwingung versetzt wird, mit einem Schwingungssensor (21), der die Amplitude einer durch die Lichtquelle (14) ausgelösten Schwingung (22) des Gewebes (19) misst, und mit einer Steuereinheit (16), die einen Relativwert (30) bildet, indem ein aktueller Messwert (27) der Amplitude mit einem Anfangsmesswert (26) der Amplitude in ein Verhältnis gesetzt wird, und die den Relativwert (30) verarbeitet, um ein Steuersignal für die Lichtquelle (14) zu erzeugen.

2. Vorrichtung nach Anspruch 1, **dadurch gekennzeichnet, dass** der Anfangsmesswert (26) der Amplitude und der aktuelle Messwert (27) der Amplitude auf die Pulsenergie normiert werden, so dass der Relativwert (30) ein normierter Relativwert ist.

3. Vorrichtung nach Anspruch 1 oder 2, **dadurch gekennzeichnet, dass** ein Zielwert für den Relativwert (30) vorgegeben ist und dass die Steuereinheit (16) die Lichtquelle (14) so ansteuert, dass der Relativwert (30) den Zielwert erreicht.

4. Vorrichtung nach Anspruch 3, **dadurch gekennzeichnet, dass** der Zielwert zwischen 1,05 und 1,5, vorzugsweise zwischen 1,1 und 1,3, weiter vorzugsweise zwischen 1,15 und 1,25 liegt.

5. Vorrichtung nach einem der Ansprüche 1 bis 4, **dadurch gekennzeichnet, dass** eine Kennlinie über der Zeit für den Relativwert (30) vorgegeben ist und dass die Steuereinheit (16) die Lichtquelle (14) so ansteuert, dass der Relativwert (30) der Kennlinie folgt.

6. Vorrichtung nach einem der Ansprüche 1 bis 5, **dadurch gekennzeichnet, dass** das Steuersignal in einen geschlossenen Regelkreis eingebunden ist, mit dem der Relativwert auf einen vorgegebenen Zielwert oder einen vorgegebenen zeitlichen Verlauf eingestellt wird.

7. Vorrichtung nach einem der Ansprüche 1 bis 6, **dadurch gekennzeichnet, dass** die Dauer einer Pause zwischen zwei aufeinanderfolgenden Lichtpulsen kleiner ist als die Relaxationszeit des Gewebes.

8. Vorrichtung nach einem der Ansprüche 1 bis 7, **dadurch gekennzeichnet, dass** der Lichtpuls (15) auf einen Spot des Gewebes gerichtet ist, wobei der Spot einen Durchmesser zwischen 0,1 mm und 10 mm, vorzugsweise zwischen 0,2 mm und 3 mm hat.

9. Vorrichtung nach einem der Ansprüche 1 bis 8, **dadurch gekennzeichnet, dass** Therapiepulse unterschiedlicher Spektralbereiche auf das Gewebe gerichtet werden.

10. Vorrichtung nach einem der Ansprüche 1 bis 9, **dadurch gekennzeichnet, dass** innerhalb des Behandlungszeitraums (31) Lichtpulse (15) in Form von Messpulsen (18) und Lichtpulse (15) in Form von Therapiepulsen (13) auf das Gewebe (19) gesendet werden, wobei der Schwingungssensor (21) die Amplitude der mit den Messpulsen (18) ausgelösten Gewebeschwingung misst und diese Messwerte in der Steuereinheit (16) verarbeitet werden.

11. Vorrichtung nach Anspruch 10, **dadurch gekennzeichnet, dass** die Therapiepulse (13) auf dem Gewebe (19) eine größere Fläche abdecken als die Messpulse (18).

12. Vorrichtung nach Anspruch 10 oder 11, **dadurch gekennzeichnet, dass** die Therapiepulse (13) in einem anderen Spektralbereich abgegeben werden als die Messpulse (18).

13. Vorrichtung nach einem der Ansprüche 1 bis 12, **dadurch gekennzeichnet, dass** die Anzahl der Lichtpulse (15) während einer Behandlung zwischen 100 und 100.000 liegt.

14. Vorrichtung nach einem der Ansprüche 1 bis 13, **dadurch gekennzeichnet, dass** der Schwingungssensor (21) dazu ausgelegt ist, den Amplitudenmesswert (27) anhand einer durch die Schwingung des Gewebes (19) ausgelösten Druckwelle (22) zu ermitteln.

15. Vorrichtung nach einem der Ansprüche 1 bis 13, **dadurch gekennzeichnet, dass** der Schwingungssensor (21) dazu ausgelegt ist, mittels eines Interferometers die Amplitude der Gewebeexpansion und -kontraktion optisch zu ermitteln.

## Claims

1. Apparatus for treating biological tissue (19), having a light source (14) in order to send a large number of light pulses (15) onto the tissue (19) within a treatment time period (31), so that the tissue (19) is set vibrating, having a vibration sensor (21), which measures the amplitude of a vibration (22) of the tissue (19) triggered by the light source (14), and having a control unit (16), which forms a relative value (30) in which a current measured value (27) of the amplitude is related to an initial measured value (26) of the amplitude, and which processes the relative value (30) in order to generate a control signal for the light source (14) .

2. Apparatus according to Claim 1, **characterized in that** the initial measured value (26) of the amplitude and the current measured value (27) of the amplitude are normalized to the pulse energy, so that the relative value (30) is a normalized relative value.

3. Apparatus according to Claim 1 or 2, **characterized in that** a target value for the relative value (30) is predefined, and **in that** the control unit (16) controls the light source (14) so that the relative value (30) reaches the target value.

4. Apparatus according to Claim 3, **characterized in that** the target value lies between 1.05 and 1.5, preferably between 1.1 and 1.3, more preferably between 1.15 and 1.25.

5. Apparatus according to one of Claims 1 to 4, **characterized in that** a characteristic curve over time is predefined for the relative value (30), and **in that** the control unit (16) controls the light source (14) so that the relative value (30) follows the characteristic curve.

6. Apparatus according to one of Claims 1 to 5, **characterized in that** the control signal is integrated into a closed control loop, with which the relative value is set to a predefined target value or a predefined time profile.

7. Apparatus according to one of Claims 1 to 6, **characterized in that** the duration of a pause between two successive light pulses is shorter than the relaxation time of the tissue.

8. Apparatus according to one of Claims 1 to 7, **characterized in that** the light pulse (15) is directed at a spot on the tissue, wherein the spot has a diameter between 0.1 mm and 10 mm, preferably between 0.2 mm and 3 mm.

9. Apparatus according to one of Claims 1 to 8, **characterized in that** therapy pulses of different spectral ranges are directed at the tissue.

10. Apparatus according to one of Claims 1 to 9, **characterized in that** within the treatment time period (31), light pulses (15) in the form of measurement pulses (18) and light pulses (15) in the form of therapy pulses (13) are sent onto the tissue (19), wherein the vibration sensor (21) measures the amplitude of the tissue vibration triggered by the measurement pulses (18) and these measured values are processed in the control unit (16) .

11. Apparatus according to Claim 10, **characterized in that** the therapy pulses (13) cover a larger area on the tissue (19) than the measurement pulses (18).

12. Apparatus according to Claim 10 or 11, **characterized in that** the therapy pulses (13) are output in a different spectral range from the measurement pulses (18).

13. Apparatus according to one of Claims 1 to 12, **characterized in that** the number of light pulses (15) during a treatment lies between 100 and 100,000.

14. Apparatus according to one of Claims 1 to 13, **characterized in that** the vibration sensor (21) is designed to determine the amplitude measured value (27) by using a pressure wave (22) triggered by the vibration of the tissue (19).

15. Apparatus according to one of Claims 1 to 13, **characterized in that** the vibration sensor (21) is designed to determine the amplitude of the tissue expansion and contraction optically by means of an interferometer.

## Revendications

1. Dispositif de traitement de tissu (19) biologique, comprenant une source de lumière (14) afin d'émettre, au sein d'une période de traitement (31), une pluralité d'impulsions de lumière (15) sur le tissu (19), de sorte que le tissu (19) est mis en oscillation, comprenant un capteur d'oscillation (21) qui mesure l'amplitude d'une oscillation (22) du tissu (19) déclenchée par la source de lumière (14), et comprenant une unité de commande (16) qui forme une valeur relative (30) en mettant la valeur mesurée actuelle (27) de l'amplitude en relation avec une valeur mesurée initiale (26) de l'amplitude, et qui traite la valeur relative (30) afin de générer un signal de commande pour la source de lumière (14).

2. Dispositif selon la revendication 1, **caractérisé en ce que** la valeur mesurée initiale (26) de l'amplitude et la valeur mesurée actuelle (27) de l'amplitude sont normalisées sur l'énergie d'impulsion, de sorte que la valeur relative (30) est une valeur relative normalisée.

3. Dispositif selon la revendication 1 ou 2, **caractérisé en ce qu'**une valeur cible est prédéfinie pour la valeur relative (30) et **en ce que** l'unité de commande (16) commande la source de lumière (14) de telle sorte que la valeur relative (30) atteint la valeur cible.

4. Dispositif selon la revendication 3, **caractérisé en ce que** la valeur cible est comprise entre 1,05 et 1,5, de préférence entre 1,1 et 1,3, encore de préférence entre 1,15 et 1,25.

5. Dispositif selon l'une des revendications 1 à 4, **caractérisé en ce qu'**une courbe caractéristique de la valeur relative (30) en fonction du temps est prédéfinie et **en ce que** l'unité de commande (16) commande la source de lumière (14) de telle sorte que la valeur relative (30) suit la courbe caractéristique.

6. Dispositif selon l'une des revendications 1 à 5, **caractérisé en ce que** le signal de commande est intégré dans un circuit de régulation en boucle fermée avec lequel la valeur relative est réglée sur une valeur cible prédéfinie ou sur un tracé dans le temps prédéfini.

7. Dispositif selon l'une des revendications 1 à 6, **caractérisé en ce que** la durée d'une pause entre deux impulsions de lumière successives est inférieure à la durée de relaxation du tissu.

8. Dispositif selon l'une des revendications 1 à 7, **caractérisé en ce que** l'impulsion de lumière (15) est dirigée sur un point du tissu, le point ayant un diamètre ente 0,1 mm et 10 mm, de préférence entre 0,2 mm et 3 mm.

9. Dispositif selon l'une des revendications 1 à 8, **caractérisé en ce que** des impulsions de thérapie de différentes plages spectrales sont dirigées sur le tissu.

10. Dispositif selon l'une des revendications 1 à 9, **caractérisé en ce que** des impulsions de lumière (15) sous la forme d'impulsions de mesure (18) et des impulsions de lumière (15) sous la forme d'impulsions de thérapie (13) sont émises sur le tissu (19) au sein de la période de traitement (31), le capteur d'oscillation (21) mesurant l'amplitude de l'oscillation du tissu déclenche avec les impulsions de mesure (18) et ces valeurs mesurées étant traitées dans l'unité de commande (16).

11. Dispositif selon la revendication 10, **caractérisé en ce que** les impulsions de thérapie (13) couvrent une surface plus grande sur le tissu (19) que les impulsions de mesure (18).

12. Dispositif selon la revendication 10 ou 11, **caractérisé en ce que** les impulsions de thérapie (13) sont délivrées dans autre plage spectrale que les impulsions de mesure (18).

13. Dispositif selon l'une des revendications 1 à 12, **caractérisé en ce que** le nombre d'impulsions de lumière (15) pendant un traitement est compris entre 100 et 100 000.

14. Dispositif selon l'une des revendications 1 à 13, **caractérisé en ce que** le capteur d'oscillation (21) est conçu pour déterminer la valeur d'amplitude (27) à l'aide d'une onde de pression (22) déclenchée par l'oscillation du tissu (19).

15. Dispositif selon l'une des revendications 1 à 13, **caractérisé en ce que** le capteur d'oscillation (21) est conçu pour déterminer optiquement l'amplitude de l'expansion et de la contraction du tissu au moyen d'un interféromètre.
